# EUROPEAN PATENT APPLICATION

(11) **EP 2 486 914 A2**
(43) Date of publication of application: **15.08.2012**
(21) Application number: 12154637.8
(22) Date of filing: 09.02.2012
(51) Int. Cl.: A61K 9/00

(54) **Stable, non-corrosive formulations for pressurized metered dose inhalers**

(30) Priority: 10.02.2011 US 201113024414
(71) Applicant: Laboratorio Pablo Cassara S.r.L., 1408 Buenos Aires (AR)
(72) Inventor: Vega, Julio César, Buenos Aires (AR)
(74) Representative: Strych, Werner Maximilian Josef

(57) **Abstract**

Pharmaceutical pressurized metered dosing inhalers (MDIs) are disclosed containing at least one dissolved halide substance, a suspended drug substance, a disolved drug substance and a co-solvent, stabilized chemically and also against metal corrosion by the addition of an acid, an unsaturated suspending agent and water.

## Description

The instant invention relates to the use of a combination of unsaturated suspending agent, water and acid to achieve stable and non-corrosive metered dose inhalers (MDI) formulations allowing use of standard AISI 316 L and coated and uncoated aluminum alloy cans without generating corrosion.

### BACKGROUND

In general for an MDI formulation to become a pharmaceutical product, it should remain physically and chemically stable throughout its shelf life. McNamara (US Patent 6,423,298) teaches MDI formulations that may contain a pharmaceutically active ingredient in suspension and one pharmaceutically active ingredient in solution. However, this patent does not teach how to get stable, non-corrosive formulations.

In the case of formulations containing at least one drug substance in suspension and at least one drug substance in solution the use of acids as stabilizers is practically unavoidable as taught by McNamara (US Patent 6,423,298). However, formulations containing acids and dissolved halides either coming from the drug substance (as is the case with Ipratropium Bromide, Fenoterol Hydrobromide and others alike) or from the acid (hydrochloric acid) are very corrosive as taught by Hoelz (US Patent No. 6,983,743). Therefore, getting a stable and non-corrosive MDI formulation able to be packaged in conventional cans is not a straightforward matter and there is no procedure known in the art to achieve this goal.

In the instant invention it was found that using a combination of certain amounts of one unsaturated suspending agent, an acid and water can surprisingly solve this difficult practical problem, using an alcohol as co-solvent as is usually the case in the art.

Several formulations have been already disclosed using alcohols as cosolvent either to dissolve drug substances or excipients (e.g. Purewal et al. US 5,776,434, Akehurst et al. US 6,893,628) and/or using acid as stabilizer of drug substances (Jager et al. US 5,955,058 and McNamara et al. US 6,423,298). However, it is most surprising to find that water can be a stabilizer for these systems in certain conditions, since it is well known in the art that water usually has a deleterious effect on suspensions as quoted by McNamara (US Patent 6,423,298): "Thus, ingress of humidity during storage or a desired increase in polarity, e.g., achieved by adding co-solvents, can have a devastating effect on the quality of the medical end product, particularly if the suspended particles have polar structure elements." Even more surprisingly this approach works even in the case of suspended polar substances with relatively high water solubility such as Salbutamol Sulfate, as can be seen in Example 1. Furthermore, McNamara (US Patent 6,423,298) mentions water as a possible co-solvent, but he does not give any example containing water and he explicitly affirms that water can have devastating effects especially when the suspended drug substance/s has/have a polar structure as is the case here. The instant invention thus promotes a contrarian teaching in the formulation of MDIs.

In order to better illustrate the innovative character of the instant invention regarding physical stability of the suspended drug substance/s, the mechanisms of suspension instability should be reviewed briefly. There are three main irreversible mechanisms that render a suspension of particles unstable:
Crystal growth: In this case, suspended particles dissolving partially in the suspending liquid recrystallize on the surface of the already existing crystals and make them grow. This enlarges crystals and lowers their therapeutic potential for inhalation because only particles having less than 10 p can be deposited in the bronchi and alveoli. Adding water to a formulation having a substance with significant water solubility could obviously provoke this sort of instability and is therefore avoided in the art as promoted by McNamara (US Patent 6,423,298)
Caking: In this case suspended particles tend to form tight aggregates, which cannot be re-suspended easily. These aggregates either clog the valve of the MDI or the orifice of the actuator or, if they become numerous, also influences the uniformity of dose, i.e., the amount of suspended drug substance varies greatly between actuations, thus rendering the formulation useless for pharmaceutical purposes. Caking is usually avoided by adding suspending agents.
   In MDIs suspending agents are usually very hydrophobic due to the generally hydrophobic nature of the suspending liquid phase. These suspending agents act by forming a hydrophobic relatively rigid sphere surrounding the suspended particle, projecting their polar groups (-COOH, -OH, ester groups, etc.) towards the surface of the suspended particle and the hydrocarbon chain towards the suspending liquid. They work particularly well when the suspending liquid is highly hydrophobic because in this case the projection of the hydrocarbon chain expands into the liquid and act as a shield so that when two particles collide they do not form tight aggregates and thus remain separated (deflocculated suspension) or form flocs (flocculated suspension). Flocs are loose aggregates having liquid phase in them and easily re-suspended.
   The use of water and a hydrophobic suspending agent is usually regarded as contradictory in the art, because it would raise the polarity of the suspending liquid phase and would be deleterious towards the formation of the "sphere" of hydrophobic suspending agent surrounding the particles. This could lead to caking.
Sticking of suspended drug/s to the can or valve walls: this effect is usually linked to the presence of water in the formulation, because water could act as a local solubilizer and stick the drug to the oxide present in the walls of aluminum or stainless steel alloys usually employed as primary package for MDIs.

In conclusion, therefore, water can foster crystal growth, caking and sticking to the walls.

In the present invention it has been found that the water and unsaturated suspending agent may build stable formulations.

In the instant invention it is disclosed that using water combined with unsaturated suspending agents and acids in certain proportions it is also possible to obtain non-corrosive formulations. Thus, regarding corrosiveness, the innovative character of the instant invention can be clearly understood, if it is taken into account that water is usually regarded as causing corrosion as well as the inclusion of an acid.

While not wishing to be limited to any particular theory, it is believed that the formulations of the instant invention are capable of achieving low corrosiveness due to the following facts:
The inclusion of an unsaturated suspending agent lowers the redox potential of the liquid phase, thus rendering it less oxidative and thus less corrosive.
The inclusion of water strongly hydrates the dissolved halides, rendering them less chemically aggressive, thus avoiding pitting and crevice corrosion. (Remark: halides in acid milieu can give rise to "pitting" corrosion or even a more critical form called "crevice corrosion" may appear. These forms of corrosions are highly localized. Furthermore, the attack of halides or other aggressive anions on intergranular areas where the Chromium content has been reduced due to the formation of Chromium Carbide could give rise to "inter-granular" corrosion.)
The inclusion of water surprisingly lowers the corrosiveness introduced by the acid component, without compromising the physical stability of the suspension.
   Furthermore, as flocculation behavior may be modified with the addition of a certain amount of water, suspension may be even more stable or easily resuspended.

Even though Hoelz (US Patent No. 6,983,743) teaches water-containing formulations these are said to be extremely corrosive requiring use of corrosive-resistant stainless steel alloys to package them. Unlike these formulations of the instant invention may be packaged into conventional cans made of aluminum alloys or stainless steel (e.g. AISI 316 L). The formulations remain stable and without any sign of corrosion for at least 18 months in zone IV b conditions (i.e. at 30 °C / 75 % RH), which are the most challenging conditions for the stability of MDIs in the whole world.

Even though the amount of unsaturated suspending agent, water, acid and co-solvent should be adjusted for each combination of pharmaceutically active ingredients, as a general rule the invention formulations comprise:
Unsaturated suspending agent: 0.001 - 0.3 %, the exact amount is approximately 1/100 to 1/10 of the amount of drug substance expressed as weight/weight percentage. The exact amount depends on the drug substance in order to get a suitable suspension, and the water content, co-solvent and acid to avoid corrosion.
Water: 0.1 - 10 %, the exact amount depends on the amount of co-solvent, the amount of unsaturated suspending agent and the amount of acid. In the case of ethanol the amount of water should be between 1/100th to 1 /5th of the amount of ethanol.
Organic Acid: 0.0001 to 0.01 % and/or mineral acid 0.000001 to 0.01 %, the exact amount depends on the acidity needed to chemically stabilize the drug substances without provoking corrosion on the can or other metal pieces in contact with the formulation.

This combination of ingredients in the instant invention allows formulation of combination of non-soluble suspended drug substances such as Salbutamol Sulfate, Budesonide, Fluticasone Propionate, Salmeterol Xinafoate or other such drugs with a dissolved halidecontaining drug substance such as: Ipratropium Bromide, Oxitropium Bromide, Tiotropium Bromide, Fenoterol Hydrobromide.

A strong argument in favor of the innovation introduced by instant invention is the fact that no CFC-free formulation of Combivent MDI (Boehringer Ingelheim Pharmaceutical Inc.'s MDI containing Albuterol Sulfate + Ipratropium Bromide Monohydrate in the strength of 100 mcg + 21 mcg per shot) is available n the market or has been submitted to FDA for approval so far, even though Boehringer Ingelheim Pharmaceuticals Inc. is the assignee of McNamara's US Patent 6,423,298 issued in 2002, where a possible formulation of Albuterol Sulfate + Ipratropium Bromide Monohydrate using HFA propellant was included as example. The McNamara's invention therefore falls short of creating a commercially feasible product, a significant drawback that has now been resolved in the present invention.

Regarding filling procedures, there are three major procedures in use at present:
Cold filling: the formulation is maintained below -40 °C, filled into cans and valve is crimped on cans afterwards.
One-step pressure filling: the formulation is filled into cans through valves already crimped onto them.
Two-step pressure filling: a concentrate consisting on co-solvent/s, surfactant/s, stabilizer/s and active ingredient/s is filled into cans, next valves are crimped onto them and propellant is filled under pressure through the already crimped valves.

The three filling procedures can be used for these formulations.

### DETAILED DESCRIPTION OF THE INVENTION

The instant invention significantly improves the formulations of MDIs. Surprisingly, inclusion of unsaturated suspending agent, water and acid in certain amounts produces stable and non-corrosive formulations with at least one suspended pharmaceutically active ingredient and at least one dissolved pharmaceutically active ingredient. The instant invention is particularly useful when there is a dissolved halide in the formulation, which, in another formulation could give rise to corrosion of the primary packaging material. The dissolved halide can come from a dissolved pharmaceutically active ingredient or from another component.

In all embodiments of the instant invention, an unsaturated suspending agent is included in formulations containing at least one of the following dissolved active ingredients: Ipratropium Bromide, Oxitropium Bromide, Tiotropium Bromide and Fenoterol Hybromide. The formulation includes a suspended drug substance such as: Salbutamol, Fenoterol, Formoterol, Budesonide, Nedocromil, Cromoglycinic acid, Salmeterol, Fluticasone and its/their salts or esters. The said formulation is packaged into appropriate stainless steel or coated or uncoated aluminum alloy cans with appropriate metering valves.

In a preferred embodiment of the instant invention, oleic acid is included in formulations containing at least one of the following dissolved active ingredients: Ipratropium Bromide, Oxitropium Bromide, Tiotropium Bromide and Fenoterol Hybromide. The formulation includes a suspended drug substance such as: Salbutamol, Fenoterol, Formoterol, Budesonide, Nedocromil, Cromoglycinic acid, Salmeterol, Fluticasone and its/their salts or esters. The said formulation is packaged into appropriate stainless steel or coated or uncoated aluminum cans with appropriate metering valves.

A particularly preferred embodiment of the instant invention contains dissolved Ipratropium Bromide with suspended Salbutamol Sulfate (also named Albuterol Sulfate) in a formulation containing between 0.0001 and 0.01 % w/w Citric Acid, water in an amount between 0.1 and 2 % w/w and Oleic Acid in an amount between 0.001 and 0.1 % packaged into AISI 316 L stainless steel cans.

In all embodiments of the instant invention, the active ingredients are present in therapeutically effective and safe concentration, so that one or more shots provide the necessary medically needed amount of drug substances to achieve effective and safe treatment.

In all embodiments of the instant invention, additional surfactants may be added. Surfactants are substances which adsorb onto the particle surface and regulate flocculation rate, floc size and weight and avoid formation of compact aggregates (caking) and recrystallization, in order to keep particle size distribution adequate for lung penetration, typically most of them smaller than 10 p. Preferred additional surfactants include Palmitic Acid, Stearic Acid, Sorbitan esters and Lecithin.

In all embodiments of the instant invention, co-solvent may be added. Preferred co-solvents include Ethyl Alcohol, Isopropyl Alcohol and other pharmaceutically acceptable Alcohols.

Co-solvent increases the solubility of the dissolved active ingredient in the propellant and, sometimes, also the solubility of the surfactant/s used.

In all embodiments of the instant invention, an optimal amount of water is added to achieve physically and chemically stable in time.

In all embodiments of the instant invention, one or more pharmaceutically acceptable propellants is/are added, selected from the group consisting of

Norflurane (HFA 134A), HFA 227 ea, Hydrocarbons or other propellants known by those skilled in the art.

In all embodiments of the instant invention, the formulation can be packaged into cans fitted with 20 to 200-microliter metering valves.

In all embodiments of the instant invention, either cold filling, one-step pressure filling or two-step pressure filling may be used to manufacture the metered dose inhalers.

### EXAMPLE 1

This example illustrates that when Oleic Acid is added, corrosion does not appear, independently of the amount of water added to the formulation. The following formulations were prepared and pressure-filled into cans onto which appropriate metering valves had been previously crimped:

**Table 1: Composition of test formulations**

| **Ingredient** | **A** | **B** | **C** | **D** |
|---|---|---|---|---|
| Salbutamol Sulfate | 120 mcg | 120 mcg | 120 mcg | 120 mcg |
| Ipratropium Bromide Monohydrate | 21 mcg | 21 m cg | 21 mcg | 21 m cg |
| Citric Acid anhydrous | 1.4525 mcg | 1.4525 mcg | 1.4525 mcg | 1.4525 mcg |
| Oleic Acid | 0.0122 mg | 0.0122 mg | 0.0122 mg | 0.0122 mg |
| Absolute Ethanol | 5.81 mg | 5.81 mg. | 5.81 mg. | 5.81 mg. |
| Water | 0 | 0.0581 mg | 0.1452 mg | 0.29 mg |
| Norflurane (HFA 134A) | s. q. 1 shot | | | |

**Table 2. The compositions of Table 1as percentage weight-by-weight (% w/w) format:**

| **Ingredient** | **A** | **B** | **C** | **D** |
|---|---|---|---|---|
| Salbutamol Sulfate | 0.207 | 0.207 | 0.207 | 0.207 |
| Ipratropium Bromide Monohydrate | 0.036 | 0.036 | 0.036 | 0.036 |
| Citric Acid anhydrous | 0.0025 | 0.0025 | 0.0025 | 0.0025 |
| Oleic Acid | 0.021 | 0.021 | 0.021 | 0.021 |
| Absolute Ethanol | 10 | 10 | 10 | 10 |
| Water | 0 | 0.1 | 0.25 | 0.5 |
| Norflurane (HFA 134A) | s. q. 100 | | | |

No corrosion was found in any sample even after 3 months at 50°C (the first month samples were stored in valve-up orientation and the 2^{nd} and 3^{rd} month in valve-down orientation in order to fully expose all inner surfaces to both gas and liquid phases).

### EXAMPLE 2

This example demonstrates how the addition of water alters the type of suspension formed, from a deflocculated or minimally flocculated one to an easily re-suspendable flocculated suspension. This means that, contrary to the teaching in the prior art, addition of water did not produce any deleterious effects in anhydrous suspensions of polar substances. In the instant invention, presence of water prevents the system from 'caking' (i.e., formation of tight aggregates, which become difficult to re-suspend by shaking).

The following formulations were tested (%w/w):

**Table 3: Compositions of formulations tested for effect of water**

| **Ingredient** | **A** | **B** | **C** | **D** | **E** | **F** | **G** | **H** | **I** |
|---|---|---|---|---|---|---|---|---|---|
| Salbutamol Sulfate | 0.207 | 0.207 | 0.207 | 0.207 | 0.207 | 0.207 | 0.207 | 0.207 | 0.207 |
| Ipratropium Bromide Monohydrat e | 0.036 | 0.036 | 0.036 | 0.036 | 0.036 | 0.036 | 0.036 | 0.036 | 0.036 |
| Citric Acid anhydrous | 0.0025 | 0.0025 | 0.0025 | 0.0025 | 0.0025 | 0.0025 | 0.0025 | 0.0025 | 0.0025 |
| Oleic Acid | 0.021 | 0.021 | 0.021 | 0.021 | 0.021 | 0.021 | 0.021 | 0.021 | 0.0021 |
| Absolute Ethanol | 10 | 10 | 10 | 10 | 10 | 10 | 10 | 10 | 10. |
| Water | 0 | 0.1 | 0.25 | 0.5 | 0.75 | 1 | 1.25 | 1.5 | 2 |
| Norflurane (HFA 134A) | s. q. 100 | | | | | | | | |

All formulations were prepared by dissolving Citric Acid, Oleic Acid in absolute Ethanol. Next Ipratropium Bromide was added and dissolved. Then stirring dispersed Salbutamol Sulfate. Finally water was added to formulations B to I.

These formulations were prepared inside transparent pressurized tubes to allow observing the sedimentation behavior and photographs were taken ten seconds after shaking. This is a relatively exaggerated time to give the patient the chance to take the medication after shaking.

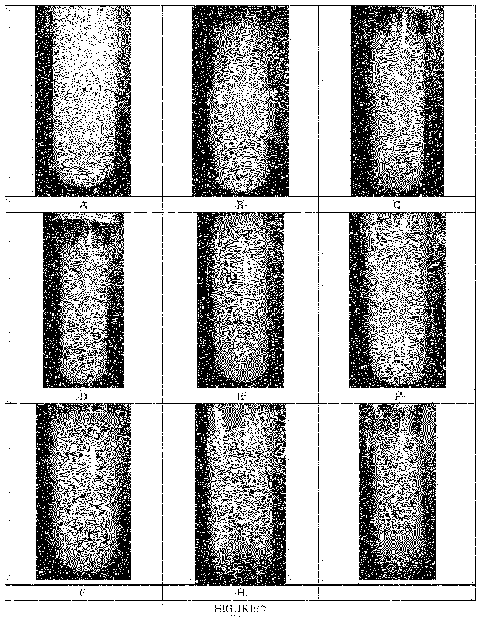

Photographs of formulations 10 seconds after shaking are shown in Figure 1.

### DESCRIPTION OF DRAWINGS

Figure 1 illustrates the photographs of the nine formulations listed in Table 3.

The following conclusions can be drawn:
Absence of added water produces a deflocculated suspension. This can be noticed in the photograph because of the turbidity of the liquid phase. This turbidity is due to the presence of isolated crystals in suspension.
Adding water from 0.1 to 1.25 % produces a flocculated suspension with relatively long sedimentation time (longer than 10 seconds). Flocculation can be clearly noticed because of the clarity of the liquid phase surrounding the flocs.
Formulations with 1.5 % w/w and 2 % w/w added water are biphasic. They form an emulsion when shaken, but separate into two phases, the upper phase being aqueous.

It is clearly demonstrated in the instant invention that there is a range of water content where suspension can be optimal, i.e. easily re-suspendable (i.e., flocculated) with an acceptable sedimentation time.

### EXAMPLE 3

This example illustrates a preferred embodiment of formulation containing one pharmaceutically active ingredient in suspension (Salbutamol Sulfate), one pharmaceutically active ingredient in solution (Ipratropium Bromide), and a dissolved halide (Bromide), using an optimized combination of unsaturated suspending agent (Oleic Acid), an acid and water.

**Table 4. Composition of a preferred embodiment.**

| **Component** | **Amount (% w/w)** |
|---|---|
| Salbutamol Sulfate | 0.207 |
| Ipratropium Bromide Monohydrate | 0.036 |
| Citric Acid (anhydrous) | 0.0025 |
| Oleic Acid | 0.021 |
| Ethanol (absolute) | 10.00 |
| Water | 0.50 |
| Norflurane (HFA 134A) | s.q. 100 g |

This formulation was prepared by dissolving Citric Acid in water, then Oleic Acid was dissolved in Ethanol and the latter solution was mixed together with the aqueous solution of Citric Acid in an airtight mixing vessel. Next Ipratropium was dissolved and then Salbutamol Sulfate was dispersed. Norflurane was added under its own pressure. This dispersion was filled under pressure into cans already fitted with valves. Units were stored for 6 months at 40 °C / 75 % RH and for 18 months at 30 °C / 75 % RH. No corrosion was found in any unit after being visually inspected after opening. In addition, all tested parameters remained within specifications, including uniformity of content, related substances and fine particle dose. Suspension stability in the metering chamber of this formulation was clearly demonstrated by leaving the MDI in the valve-down position (as the patient does) during more than 10 seconds. The metered shot Salbutamol Sulfate content is as follows, taking as 100 % of the value obtained when actuating immediately):
After 15 seconds: 106.7 %
After 30 seconds: 107.4 %

### EXAMPLE 4

The following formulation filled by one-step pressure filling into AISI 316 L cans fitted with 50-microliter metering valve is another useful example of the invention:

**Table 4. Preferred embodiment of one-step pressure filling formulation.**

| **Component** | **Amount (% w/w)** |
|---|---|
| Fluticasone Propionate | 0.439 |
| Formoterol Fumarate Dihydrate | 0.0105 |
| Oleic Acid | 0.04 |
| Hydrochloric acid 36 % w/w | 0.0024 (*) |
| Ethanol (absolute) | 12.00 |
| Water | 0.10 |
| Norflurane (HFA 134A) | s.q. 100 g |

| | |
|---|---|
| (*) The exact amount to obtain an apparent pH of 2.5 to 5.0 is added. HCl 1 M may be added to facilitate the operation if necessary. | |

The amounts of Fluticasone Propionate and Formoterol Fumarate Dihydrate correspond to 250 mcg and 6 mcg per shot delivered from the valve. These are the metered dose of each drug substance in use in Europe at the moment. The formulation is prepared in a stirred airtight mixing vessel by dissolving Oleic Acid and Water in anhydrous Ethanol. Next Fluticasone Propionate is added under stirring. Next Hydrochloric acid 36 % w/w is added and finally Formoterol Fumarate Dihydrate is dissolved under stirring. Norflurane added into the airtight mixing vessel. The solution is pressure filled into cans already fitted with a 50-mcl metering valve.

## Claims

1. A corrosion-resistant, pharmaceutically-stable pressurized metered dose inhaler composition comprising:
at least one suspended pharmaceutical active ingredient;
at least one dissolved pharmaceutical active ingredient;
a dissolved halide;
at least one co-solvent;
at least one propellant;
water in the range 0.1 to 10 % w/w;
an organic acid in the range 0.0001 to 0.01 % w/w; and
an unsaturated suspending agent in the range 0.01 to 0.3 % w/w.

2. The composition of claim 1 wherein said halide comes from said pharmaceutical active ingredients.

3. The composition of claim 1 wherein said organic acid is replaced by a mineral acid in the range 0.000001 to 0.01 % w/w.

4. The composition of claim 1 wherein said composition additionally contains a mineral acid in the range 0.000001 to 0.01 % w/w.

5. The composition of claim 1 wherein said composition is packaged in cans fitted with a metering valve delivering between 20 and 200 microliters of said composition per shot.

6. The composition of claim 1 wherein said composition is packaged in cans made of materials selected from a group comprising AISI 316L stainless steel, uncoated aluminum alloy and coated aluminum alloy.

7. The composition of claim 1 wherein said dissolved pharmaceutical active ingredient is selected from a group consisting of Fenoterol Hydrobromide, Ipratropium Bromide, Oxitropium Bromide, Tiotropium Bromide, and Formoterol Fumarate.

8. The composition of claim 1 wherein said suspended pharmaceutical active ingredient is selected from a group consisting of Salbutamol Sulfate, R-Salbutamol Sulfate, Budesonide, Ciclosonide, Fluticasone Propionate, Fluticasone Fumarate, Salmeterol Xinafoate, and Formoterol Fumarate.

9. The composition of claim 1 wherein said suspended pharmaceutical active ingredient is Salbutamol Sulfate and dissolved pharmaceutical active ingredient is Ipratropium Bromide Monohydrate.

10. The composition of claim 1 wherein said co-solvent is ethanol or isopropanol.

11. The composition of claim 1 wherein said propellant is selected from a group consisting of fluorinated hydrocarbons HFA 134A, HFA 227ea or a mixture thereof.

12. The composition of claim 1 wherein said propellant is a hydrocarbon.

13. The composition of claim 1 wherein said propellant is a mixture of said hydrocarbon and said fluorinated hydrocarbon.

14. A method of producing a stabled and non-corrosive formulation for metered dose inhaler comprising addition of an unsaturated suspending agent in the range fo 0.01 % w/w to 0.3 % w/w and an amount of water in the range 0.1 % w/w to 10% w/w.

15. The method of claim 14 wherein said formulation is stable for at least six months at 40°C/75% RH storage condition and 2 years at 30°C/75% RH storage
